# EUROPEAN PATENT APPLICATION

(11) **EP 4 410 371 A1**
(43) Date of publication of application: **07.08.2024**
(21) Application number: 23154936.1
(22) Date of filing: 03.02.2023
(51) Int. Cl.: A61P 31/04, C07K 16/12

(54) **THERAPEUTIC VHH ANTIBODIES AGAINST ALPHA-HEMOLYSIN FROM STAPHYLOCOCCUS AUREUS**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention pertains in the fields of antibody technology, medicine, pharmacology, infection biology, and medical diagnostics. More specifically, the present disclosure provides VHH antibodies that prevent membrane-binding and/or oligomerization of and hemolysis by *S*. *aureus* alpha-hemolysin (HLA).

## Description

### Field of the invention

The present invention pertains in the fields of antibody technology, medicine, pharmacology, infection biology, and medical diagnostics. More specifically, the present disclosure provides VHH antibodies that prevent membrane-binding and/or oligomerization of and hemolysis by *S*. *aureus* alpha-hemolysin (HLA).

### Background

*Staphylococcus aureus* is one of the most prevalent bacterial pathogens, causing a myriad of diseases, ranging from superficial skin infections to life-threatening pneumonia, bacteremia, and sepsis. In developed countries, *S. aureus* is the leading cause of blood infections and causes the highest mortality. In fact, in the US, annual deaths from *S. aureus* constituted more than that caused by acquired immune deficiency syndrome (AIDS), tuberculosis, and viral hepatitis combined (Cheung et al., 2021). The lack of an approved vaccine and complicated treatments make S. *aureus* a major global healthcare problem.

S. *aureus* is a commensal bacterium, with an estimated 30% of the population being carriers (Tong et al., 2015; Saleh et al., 2022). However, it can readily cause infections upon injuries of the skin or mucosal membranes. The human immune system can normally eliminate invading bacteria within minutes (using complement, macrophages, and upon repeat contact also, antibodies). Virulent bacteria, however, employ virulence factors that allow to evade the immune response. Virulence factors can generally be classified as surface and extracellular secreted proteins; they include bacterial toxins, complement-inactivating factors, inhibitors of phagocytosis, or proteases that cleave immunoglobulins or extracellular matrix. Unlike other bacteria, which employs only one or a few, *S. aureus* harbors > 20 virulence factors to promote its pathogenicity (Cheung et al., 2021).

Antibiotics are key for treating bacterial infections, yet the rise of antibiotic-resistant infections complicates the therapies. *S. aureus* infections are particularly challenging due to frequently occurring multi-drug resistance in *S. aureus* isolates. Methicillin-resistant *S. aureus* (MRSA), is the most commonly identified antibiotic-resistant pathogen worldwide. MRSA-infected patients stay longer in the hospital, incur higher treatment costs, and show higher mortality (Ippolito et al., 2010). The first MRSA strain was characterized in 1961, only one year after methicillin was approved for clinical treatments (Ippolito et al., 2010; Turner et al., 2019). Since then, a plethora of strains resistant to an increasing number of antibiotics has been reported. Some clinical isolates even showed resistance to ≥ 10 antibiotic classes (Saleh et al., 2022). New antibiotics for MRSA and, more generally, for *S. aureus* therapy have been developed or are in the pipeline. Currently, vancomycin is used as the antibiotic of last resort in non-critically ill patients. (Cheung et al., 2021). Yet, although not widespread, vancomycin-resistant S. *aureus* isolates are already being identified. Likewise, an increasing number of vancomycin treatments are failing (Lodise et al., 2008; Ye et al., 2020).

Another shortcoming in antibiotic therapy is that even if the treatment is immediately effective, it cannot remove already circulating bacterial toxins from the body. Antibiotics might even enhance toxin release. Therefore, there is a need for alternative treatment strategies that target virulence factors. One such "Anti-toxin treatment" approach is to use antibodies to neutralize virulence factors, which should stop toxic effects, "disarm" the pathogen and allow the immune system to clear the infection.

Alpha-hemolysin (HLA) or alpha-toxin is the most important and best-known virulence factor of *S. aureus.* HLA is a 33 kDa secreted precursor protein that is found in > 95% of *S. aureus* isolates (Kielian et al., 2001). Upon binding to target membranes, HLA oligomerizes into a heptamer and forms a 40 Å wide pore. Such a pore causes leakage of ions and other small molecules, destabilizes the membranes, and leads to cell lysis. HLA mainly targets leukocytes for evasion from the host cell defense system, and erythrocytes making the otherwise limiting iron available for bacterial growth. In epithelial and endothelial cells, HLA interacts with ADAM10, a cell surface receptor with metalloprotease and E-cadherin domains, whose activation breaks adherens junctions and, in turn, allows bacteria to leave the bloodstream and invade soft tissues.

The direct contribution of HLA in sepsis, pneumonia and skin lesions has been welldocumented in various murine models (Kielian et al., 2001; Bubeck Wardenburg et al., 2007). In addition, in a rat model of pneumonia, strain pathogenicity has been highly correlated with elevated HLA expression (Montgomery et al., 2008). Therefore, HLAtargeted strategies, either by interfering with its expression level or by neutralizing the already circulating toxin, offer promising solutions *en route* to developing new (passive immunization) therapies against *S*. *aureus* infections.

### Summary of the invention

The present disclosure provides single-domain VHH antibodies directed to S. *aureus* alpha-hemolysin (HLA). They recognize and/or prevent membrane-binding and/or oligomerization of and hemolysis by *S. aureus* HLA and are suitable for diagnostic, prophylactic and/or therapeutic applications.

In certain embodiments, these VHH antibodies are in monovalent form, e.g., as a single VHH domain or as a fusion to a heterologous protein such as serum albumin useable in a wide range of formats. In certain embodiments, The VHH antibodies are in multivalent form, e.g., as bivalent Fc-fusion. The use in a monovalent format is possible due to their very high affinity. In certain embodiments, the VHH antibodies have - in the monovalent format - a target affinity in picomolar or even low picomolar range. In certain embodiments, the VHH antibodies neutralize HLA with high potency.

A first aspect of the present disclosure is a VHH antibody directed against *S. aureus* HLA as described herein. Preferred VHH antibodies of the present invention, their designations, binding characteristics, i.e., HLA epitope, HLA affinity, and HLA neutralization as well as their thermostabilities are listed in the following Table 1:

### Table 1

**Table 1: Anti-HLA VHH antibodies, their thermostability, affinities for HLA (expressed as K_{D} values) and inhibitory properties.**

| **VHH** | **Class** | **Epitope** | **Thermostability** | **HLA affinity (pM)** | **Inhibition of hemolysis** |
|---|---|---|---|---|---|
| Ma7A07 | Ma7A07 | 1 | >95 | ≤10 | Yes |
| Ma7A08 | Ma7A07 | 1 | >95 | ≤10 | Yes |
| Ma7B03 | Ma7A07 | 1 | >95 | ≤10 | Yes |
| Ma7B12 | Ma7A07 | 1 | >95 | ≤10 | Yes |
| Ma7B01 | Ma7B01 | 2 | 39 | 50 | Yes |
| Ma7E01 | Ma7B01 | 2 | | ≤10 | Yes |
| Ma7C02 | Ma7B01 | 2 | 57 | 90 | Yes |
| Ma7F02 | Ma7F02 | 2 | 61 | 40 | Yes |
| Ma7D06 | Ma7D06 | 2 | 51 | 60 | Yes |
| Ma7C11 | Ma7C11 | 2 | 67 | 300 | Yes |
| Ma8E03 | Ma8E03 | 1 | >95 | ≤10 | Yes |
| Ma8G02 | Ma8E03 | 1 | >95 | ≤10 | Yes |
| Ma8H03 | Ma8E03 | 1 | >95 | ≤10 | Yes |
| Ma8A05 | Ma8A05 | 4 | 44 | 600 | Yes |
| Ma8D05 | Ma8A05 | 4 | 44 | 1000 | Yes |
| Ma8D07 | Ma8D07 | 3 | 50 | 800 | Yes |
| Ma8D08 | Ma8D07 | 3 | 48 | 1000 | Yes |

A list of complete VHH sequences of VHH antibodies is shown at the end of the specification.

A further aspect of the present invention relates to a set of two or more different VHH antibodies, wherein at least one VHH antibody is as described above.

A VHH antibody described above is suitable for use in medicine, e.g., human medicine, particularly for use in therapy, e.g., in the prevention, treatment and/or mitigation of a condition caused by, associated with and/or accompanied by an infection with HLA-positive *S. aureus,* particularly by an infection with a drug-resistant HLA-positive *S. aureus,* or in diagnostics, e.g., for detecting *S. aureus* HLA in a patient sample, e.g., in a body fluid or tissue sample, or in research.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for the recombinant production of a VHH antibody as described above, comprising cultivating a cell or an organism as described above in a suitable medium and obtaining the VHH antibody from the cell or organism or from the medium.

Still a further aspect of the invention relates to a method for the prevention, treatment and/or mitigation of a condition caused by, associated with and/or accompanied by an infection with HLA-positive *S. aureus,* particularly by an infection with a drug-resistant HLA-positive *S. aureus,* comprising administering an effective dose of the VHH antibody as described above or the set of at least two different VHH antibodies as described above to a subject in need thereof, particularly to a human subject suffering from a disorder caused by, associated and/or accompanied by an infection with HLA-positive *S. aureus.*

A non-limitative list of VHH antibodies of the present invention and their CDR sequences is shown in the following Table 2:

### Table 2

**Table 2: Anti-HLA VHH antibodies and their CDR regions. Note that CDRs include here also variable residues that flank the actual CDR loops.**

| VHH | SEQ ID NO. | CDR1 | SEQ ID NO. | CDR2 | SEQ ID NO. | CDR3 | SEQ ID NO. |
|---|---|---|---|---|---|---|---|
| Ma7A07 | 3 | SGFTLGDYTLG | 4 | SSIASSALSIYIADS | 5 | RGVDSWRWVPSAMDLW | 6 |
| Ma7A08 | 7 | SGFTLGDYTLG | 8 | SSIASSVLSVYIADS | 9 | RGVDSWRWVPSAMDLW | 10 |
| Ma7B03 | 11 | SGFTLSNYYVG | 12 | SSIGSSDLSIYIADS | 13 | RGTDSWRWVPSAMDLW | 14 |
| Ma7B12 | 15 | SGFTLGDYTLG | 16 | SSIASSALSIYIADS | 17 | RGIDNWRWVPSAMDLW | 18 |
| Ma7B01 | 19 | SGRTFSNYAMG | 20 | AVINQIGDSTWYPDF | 21 | ATDPRSYWRIWPHEYGYW | 22 |
| Ma7C02 | 23 | SGRTFSDYAMG | 24 | AVITRSGDSTYYPDS | 25 | ATDPTNYWRIWEHEFDYW | 26 |
| Ma7E01 | 27 | SERTFSNYAMG | 28 | AVINPIGEETWYPDF | 29 | ATDPRSYWRIWEHEFDYW | 30 |
| Ma7F02 | 31 | SGSIDSLHTMG | 32 | AVVSWSGGNTYYPDY | 33 | AESGSGNYWKIWEHEFDSW | 34 |
| Ma7D06 | 35 | SGSISGIDTMA | 36 | AVIKWAAGSTWYPDF | 37 | AAAGEKYYYRLFPYEYDYW | 38 |
| Ma7C11 | 39 | SGSISSVNGMG | 40 | AQVSLLDSSTYYADS | 41 | ATKLRIGTAFSSEYDYW | 42 |
| Ma8E03 | 43 | SGSSLDHYVIG | 44 | SCISRSGGSTNYADS | 45 | AQRNLGNFCVLGWLEYDDW | 46 |
| Ma8G02 | 47 | SGSSLDHYVIG | 48 | SCISRSGGSTNYADS | 49 | AQRNLGNFCVLGWVEYDDW | 50 |
| Ma8H03 | 51 | SGSSLDHYVIG | 52 | SCISSGGSTNYADS | 53 | AQRNLGNFCVLGWLEYDDW | 54 |
| Ma8A05 | 55 | SPASGFTLDLYTIA | 56 | SAISLSDEITYYSDA | 57 | IGAFDFILKEADADYW | 58 |
| Ma8D05 | 59 | SPASGFTLDLYTIA | 60 | SAISLSDEITYYSDT | 61 | IGAFDFILKEADADYW | 62 |
| Ma8D07 | 63 | SESIYKLNAMG | 64 | TTISSGGSTFYTDP | 65 | ANPTYNHYSAR | 66 |
| Ma8D08 | 67 | SESIYKLNAMG | 68 | TTISSGGSTFYTDP | 69 | ANPTYNHYSAR | 70 |

### Embodiments of the invention

In the following, specific embodiments of the invention are disclosed as follows:
1. A VHH antibody recognizing an *S. aureus* alpha-hemolysin (HLA).
2. The VHH antibody of embodiment 1, which competes with the VHH antibody Ma7A07 having a VHH sequence as shown in SEQ. ID NO: 3 for the binding to HLA.
3. The VHH antibody of embodiment 1, which competes with the VHH antibody Ma7B01 having a VHH sequence as shown in SEQ. ID NO: 19 for the binding to HLA.
4. The VHH antibody of embodiment 1, which competes with the VHH antibody Ma8A05 having a VHH sequence as shown in SEQ. ID NO: 55 for the binding to HLA.
5. The VHH antibody of embodiment 1, which competes with the VHH antibody Ma8D07 having a VHH sequence as shown in SEQ. ID NO: 63 for the binding to HLA.
6. A VHH antibody recognizing an *S. aureus* HLA polypeptide, comprising
   (a) a CDR3 sequence as shown in SEQ. ID NO: 6, 10, 14, 18, 22, 26, 30, 34, 38, 42, 46, 50, 54, 58, 62, 66 or 70;
   (b) a CDR3 sequence, which has an identity of at least 80%, at least 90% or at least 95% to a CDR3 sequence of (a), or
   (c) a VHH antibody, which competes with the VHH antibody Ma7A07 having a VHH sequence as shown in SEQ. ID NO: 3 for the binding to HLA, or
      which competes with the VHH antibody Ma7B01 having a VHH sequence as shown in SEQ. ID NO: 19 for the binding to HLA, or
      which competes with the VHH antibody Ma8A05 having a VHH sequence as shown in SEQ. ID NO: 55 for the binding to HLA, or
      which competes with the VHH antibody Ma8D07 having a VHH sequence as shown in SEQ. ID NO: 63 for the binding to HLA.
7. The VHH antibody of any of the preceding embodiments, comprising
   (a) a combination of CDR1, CDR2 and CDR3 sequences as shown in SEQ. ID NO: 4-6, 8-10, 12-14, 16-18, 20-22, 24-26, 28-30, 32-34, 36-38, 40-42, 44-46, 48-50, 52-54, 56-58, 60-62, 64-66, or 68-70,
   (b) a combination of CDR1, CDR2 and CDR3 sequences which has an identity of at least 80%, at least 90% or at least 95% to a combination of CDR1, CDR2 and CDR3 sequences of (a), or
   (c) a VHH antibody, which competes with the VHH antibody Ma7A07 having a VHH sequence as shown in SEQ. ID NO: 3 for the binding to HLA, or
      which competes with the VHH antibody Ma7B01 having a VHH sequence as shown in SEQ. ID NO: 19 for the binding to HLA, or
      which competes with the VHH antibody Ma8A05 having a VHH sequence as shown in SEQ. ID NO: 55 for the binding to HLA, or
      which competes with the VHH antibody Ma8D07 having a VHH sequence as shown in SEQ. ID NO: 63 for the binding to HLA.
8. The VHH antibody of any one of the preceding embodiments, comprising
   (a) a VHH sequence as shown in SEQ. ID NO: 3, 7, 11, 15, 19, 23, 27, 31, 35, 39, 43, 47, 51, 55, 59, 63 or 67;
   (b) a sequence, which has an identity of at least 80%, at least 90%, at least 95% or at least 99% to a VHH sequence of (a), or
   (c) a VHH antibody, which competes with the VHH antibody Ma7A07 having a VHH sequence as shown in SEQ. ID NO: 3 for the binding to HLA, or
      which competes with the VHH antibody Ma7B01 having a VHH sequence as shown in SEQ. ID NO: 19 for the binding to HLA, or
      which competes with the VHH antibody Ma8A05 having a VHH sequence as shown in SEQ. ID NO: 55 for the binding to HLA, or
      which competes with the VHH antibody Ma8D07 having a VHH sequence as shown in SEQ. ID NO: 63 for the binding to HLA.
9. The VHH antibody of any one of the preceding embodiments, wherein the binding affinity expressed as dissociation constant K_{D} to HLA is about 1 nM or less, about 0.5 nM or less, or about 0.1 nM or less.
10. The VHH antibody of any one of the preceding embodiments, which neutralizes HLA, particularly by inhibiting HLA-induced hemolysis of erythrocytes.
11. The VHH antibody of embodiment 10, which neutralizes HLA. or less.
12. The VHH antibody of any one of the preceding embodiments, which is stable, particularly thermostable, or hyperthermostable.
13. The VHH antibody of embodiment 12, which has a melting temperature of at least about 40°C, of at least about 50°C, of at least about 60°C, of at least 80°C or of at least about 95°C when measured under non-reducing conditions.
14. The VHH antibody of embodiment 12 or 13, which has an aggregation temperature of at least about 40°C, of at least about 50°C, of at least about 60°C, of at least 70°C or of at least about 80°C when measured under non-reducing conditions.
15. The VHH antibody of any one of embodiments 1-14, which is selected from VHH antibody Ma7A07 having a VHH sequence as shown in SEQ. ID NO: 3 or a VHH antibody, which is a variant thereof.
16. The VHH antibody of any one of embodiments 1-14, which is selected from VHH antibody Ma7A08 having a VHH sequence as shown in SEQ. ID NO: 7 or a VHH antibody, which is a variant thereof.
17. The VHH antibody of any one of embodiments 1-14, which is selected from VHH antibody MaB03 having a VHH sequence as shown in SEQ. ID NO: 11 or a VHH antibody, which is a variant thereof.
18. The VHH antibody of any one of embodiments 1-14, which is selected from VHH antibody Ma7B12 having a VHH sequence as shown in SEQ. ID NO: 15 or a VHH antibody, which is a variant thereof.
19.The VHH antibody of any one of embodiments 1-14, which is selected from VHH antibody Ma7B01 having a VHH sequence as shown in SEQ. ID NO: 19 or a VHH antibody, which is a variant thereof.
20. The VHH antibody of any one of embodiments 1-14, which is selected from VHH antibody Ma7C02 having a VHH sequence as shown in SEQ. ID NO: 23 or a VHH antibody, which is a variant thereof.
21. The VHH antibody of any one of embodiments 1-14, which is selected from VHH antibody Ma7E01 having a VHH sequence as shown in SEQ. ID NO: 27 or a VHH antibody, which is a variant thereof.
22. The VHH antibody of any one of embodiments 1-14, which is selected from VHH antibody Ma7F02 having a VHH sequence as shown in SEQ. ID NO: 31 or a VHH antibody, which is a variant thereof.
23. The VHH antibody of any one of embodiments 1-14, which is selected from VHH antibody Ma7D06 having a VHH sequence as shown in SEQ. ID NO: 35 or a VHH antibody, which is a variant thereof.
24. The VHH antibody of any one of embodiments 1-14, which is selected from VHH antibody Ma7C11 having a VHH sequence as shown in SEQ. ID NO: 39 or a VHH antibody, which is a variant thereof.
25. The VHH antibody of any one of embodiments 1-14, which is selected from VHH antibody Ma8E03 having a VHH sequence as shown in SEQ. ID NO: 43 or a VHH antibody, which is a variant thereof.
26. The VHH antibody of any one of embodiments 1-14, which is selected from VHH antibody Ma8G02 having a VHH sequence as shown in SEQ. ID NO: 47 or a VHH antibody, which is a variant thereof.
27. The VHH antibody of any one of embodiments 1-14, which is selected from VHH antibody Ma8H03 having a VHH sequence as shown in SEQ. ID NO: 51 or a VHH antibody, which is a variant thereof.
28. The VHH antibody of any one of embodiments 1-14, which is selected from VHH antibody Ma8A05 having a VHH sequence as shown in SEQ. ID NO: 55 or a VHH antibody, which is a variant thereof.
29. The VHH antibody of any one of embodiments 1-14, which is selected from VHH antibody Ma8D05 having a VHH sequence as shown in SEQ. ID NO: 59 or a VHH antibody, which is a variant thereof.
30. The VHH antibody of any one of embodiments 1-14, which is selected from VHH antibody Ma8D07 having a VHH sequence as shown in SEQ. ID NO: 63 or a VHH antibody, which is a variant thereof.
31. The VHH antibody of any one of embodiments 1-14, which is selected from VHH antibody Ma8D08 having a VHH sequence as shown in SEQ. ID NO: 67 or a VHH antibody, which is a variant thereof.
32. The VHH antibody of any one of the preceding embodiments, which is non-glycosylated, or which is glycosylated.
33. The VHH antibody of any one of the preceding embodiments, which is produced in a bacterium, e.g., *E*. *coli,* or in a yeast, e.g., *Pichia pastoris,* or in a human or animal cell, e.g., an insect cell or a mammalian cell.
34. The VHH antibody of any one of the preceding embodiments, which is in a monovalent format.
35. The VHH antibody of any one of embodiments 1-33, which is in a multimeric format.
36. The VHH antibody of embodiment 35, which is in a dimeric format.
37. The VHH antibody of embodiment 35, which is in a homodimeric or in a heterodimeric format.
38. The VHH antibody of any one of the preceding embodiments, which is covalently or non-covalently conjugated to a heterologous moiety, e.g., a labeling group, a capture group, or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.
39. The VHH antibody of any one of the preceding embodiments, which is fused to a heterologous polypeptide moiety, or which is fused to an Fc Fragment, or which is fused to serum albumin or an albumin-binding moiety.
40. The VHH antibody of any one of the preceding embodiments, which is conjugated to one or several non-peptidic polymer moieties, preferably hydrophilic polymer moieties, such as polyethylene glycol (PEG).
41.A set of two or more different VHH antibodies recognizing HLA, comprising at least one VHH antibody of any of embodiments 1-40, particularly a VHH antibody in a monovalent format.
42. The set of embodiment 41 comprising at least two VHH antibodies selected from at least two different VHH antibody classes (i), (ii), (iii) and (iv) as follows:
   (i) a VHH antibody which is selected from VHH antibody Ma7A07 having a VHH sequence as shown in SEQ. ID NO: 3, or a VHH antibody, which competes with the VHH antibody Ma7A07 for the binding to HLA;
   (ii) a VHH antibody which is selected from VHH antibody Ma7B01 having a VHH sequence as shown in SEQ. ID NO: 19, or a VHH antibody, which competes with the VHH antibody Ma7B01 for the binding to HLA;
   (iii) a VHH antibody which is selected from VHH antibody Ma8A05 having a VHH sequence as shown in SEQ. ID NO: 55, or a VHH antibody which competes with the VHH antibody Ma8A05 for the binding to HLA; and
   (iv) a VHH antibody which is selected from VHH antibody Ma8D07 having a VHH sequence as shown in SEQ. ID NO: 63, or a VHH antibody which competes with the VHH antibody Ma8D07 for the binding to HLA.
43.The VHH antibody of any one of embodiments 1-40 or the set of embodiment 41 or 42 for use in medicine, particularly for use in therapy or diagnostics.
44.The VHH antibody of any one of embodiments 1-40 or the set of embodiment 41 or 42 for use in the prevention, treatment and/or mitigation of a condition caused by, associated with and/or accompanied by an infection with an HLA-positive *S. aureus.*
45. The VHH antibody of any one of embodiments 1-40 or the set of embodiment 41 or 42 for the use of embodiment 43 or 44 wherein the *S. aureus* is a drug-resistant HLA-positive *S. aureus.*
46. The VHH antibody of any one of embodiments 1-40 or the set of embodiment 41 or 42 for the use of embodiment 44 or 45, wherein the condition is selected from a skin infection, pneumonia, bacteremia, sepsis, meningitis, osteomyelitis, and/or endocarditis.
47. The VHH antibody of any one of embodiments 1-40 or the set of embodiment 41 or 42 for the use of any one of embodiments 43-46 in a human subject.
48.The VHH antibody of any one of embodiments 1-40 or the set of embodiment 41 or 42 for the use of any one of embodiments 43-47, which is administered locally.
49. The VHH antibody of any one of embodiments 1-40 or the set of embodiment 41 or 42 for the use of any one of embodiments 43-47, which is administered systemically.
50. The VHH antibody of any one of embodiments 1-40 or the set of embodiment 41 or 42 for the use of any one of embodiments 43-49, which is administered topically, parenterally, e.g., intravenously, pulmonally, e.g., by inhalation, or nasally, e.g., by spraying.
51. The VHH antibody of any one of embodiments 1-40 or the set of embodiment 41 or 42 for the use of any one of embodiments 43-50, which is administered as a monotherapy.
52. The VHH antibody of any one of embodiments 1-40 or the set of embodiment 41 or 42 for the use of any one of embodiments 43-50, which is administered sequentially and/or simultaneously as a combination therapy with at least one further active agent.
53.The VHH antibody of any one of embodiments 1-40 or the set of embodiment 41 or 42 for the use of embodiment 52, which is administered in combination with an antibiotic against *S. aureus,* e.g., vancomycin, and optionally with a further antibiotic against a different bacterium.
54.A pharmaceutical composition comprising as an active agent a VHH antibody of any one of embodiments 1-40 or the set of embodiment 41 or 42, and a pharmaceutically acceptable carrier.
55.A nucleic acid molecule encoding a VHH antibody or a subunit of a VHH containing subunit according to any one of embodiments 1-40, preferably in operative linkage with a heterologous expression control sequence.
56.A vector comprising a nucleic acid molecule according to embodiment 55.
57.A recombinant cell or non-human organism transformed or transfected with a nucleic acid molecule according to embodiment 55 or a vector according to embodiment 56.
58.The cell or organism of embodiment 57, which is selected from a bacterium such as *E*. *coli Bacillus sp.,* a unicellular eukaryotic organism, e.g., yeast such as *Pichia pastoris,* or *Leishmania,* an insect cell, a mammalian cell, or a plant cell.
59.A method for recombinant production of a VHH antibody of any one of embodiments 1-40, comprising cultivating a cell or an organism of embodiment 57 or 58 in a suitable medium and obtaining the VHH antibody from the cell or organism or from the medium.
60. The method of embodiment 59, comprising cultivating a yeast such as *Pichia pastoris* and obtaining the VHH antibody from the medium.
61. Use of the VHH antibody of any one of embodiments 1-40 or the set of embodiment 41 or 42 for detecting HLA in a sample.
62. The use of embodiment 61, wherein the sample is a biological sample, e.g., a body fluid such as saliva, sputum, a lung lavage, a swab, blood, serum or plasma, a stool sample, a tissue sample, or a biopsy sample.
63. The use of embodiment 61 or 62, wherein the detection comprises the binding of at least 2 different VHH antibodies to an HLA molecule.
64. The use of embodiment 63, wherein the detection comprises the binding of at least 2 different VHH antibodies, e.g., 2, 3, or 4 VHH antibodies each directed against a different epitope, to an HLA molecule.
65. The use of any one of embodiments 61-64, wherein the detection comprises a sandwich assay, e.g., a sandwich ELISA.
66. The use of any one of embodiments 61-65, wherein the detection comprises a prior HLA enrichment step.
67.A method for the prevention, treatment and/or mitigation of a condition caused by, associated with and/or accompanied by an infection with an HLA-positive *S. aureus* comprising administering an effective dose of the VHH antibody of any one of embodiments 1-40, the set of embodiment 41 or 42, or the pharmaceutical composition of embodiment 54 to a subject in need thereof, particularly to a human subject.

### Description of the Invention

### VHH antibodies

The present invention relates to a VHH antibody recognizing an S. *aureus* alpha-hemolysin (HLA).

The VHH antibody may be a monovalent heavy chain-only antibody comprising a CDR1 domain, a CDR2 domain and a CDR3 domain linked by framework regions including, but not being limited to, whole VHH antibodies, e.g., native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

The VHH antibody of the present invention may be glycosylated or non-glycosylated.

There are several methods known in the art for determining the CDR sequences of a given antibody molecule, but there is no standard unequivocal method. Determination of CDR sequences from antibody heavy chain variable regions can be made according to any method known in the art, including, but not limited to, the methods known as Kabat, Chothia, and IMGT. A selected set of CDRs may include sequences identified by more than one method, namely, some CDR sequences may be determined using Kabat and some using IMGT, for example. According to some embodiments of the present invention, the CDR sequences of a VHH variable region are determined using the Kabat method. CDRs may also be defined through a multiple alignment (with many other VHH antibodies), to identify the hot-spots of variability and relate them to a standard VHH antibody structure. It is also possible to define CDRs by analyzing the structure of the VHH antibody and deciding what is a loop and what is the antibody's scaffold. In some cases, CDR-adjacent residues are also variable, and are therefore included in the CDR definition.

The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing.

According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which competes with a specific VHH antibody disclosed herein for the binding to an *S. aureus* HLA polypeptide. In certain embodiments, a competing VHH antibody binds the same or an overlapping epitope on the *S. aureus* HLA polypeptide. For example, the invention refers to a VHH antibody, which competes with a reference antibody, e.g., VHH antibody Ma7A07 (binding to epitope 1 on HLA), with VHH antibody Ma7B01 (binding to epitope 2 on HLA), VHH antibody Ma8A05 (binding to epitope 4 on HLA), or VHH antibody Ma8D07 (binding to epitope 3 on HLA). Competition may be determined by label-free biolayer interferometry performed as a cross-competition or epitope binning assay using a label-free detection system, e.g., an Octet^{®} system from Sartorius, according to the manufacturer's instructions.

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In further particular embodiments, the VHH antibody is selected from antibody Ma7A07 having a VHH sequence as shown in SEQ. ID NO: 3 or a VHH antibody, which is a variant thereof. In certain embodiments, 1, 2, 3, 4, 5, 6, 7, 8, 9 or 10 amino acids of SEQ. ID NO: 3 are replaced by another amino acid.

In further particular embodiments, the VHH antibody is selected from antibody Ma7A08 having a VHH sequence as shown in SEQ. ID NO: 7 or a VHH antibody, which is a variant thereof. In certain embodiments, 1, 2, 3, 4, 5, 6, 7, 8, 9 or 10 amino acids of SEQ. ID NO: 7 are replaced by another amino acid.

In further particular embodiments, the VHH antibody is selected from antibody Ma7B03 having a VHH sequence as shown in SEQ. ID NO: 11 or a VHH antibody, which is a variant thereof. In certain embodiments, 1, 2, 3, 4, 5, 6, 7, 8, 9 or 10 amino acids of SEQ. ID NO: 11 are replaced by another amino acid.

In further particular embodiments, the VHH antibody is selected from antibody Ma7B12 having a VHH sequence as shown in SEQ. ID NO: 15 or a VHH antibody, which is a variant thereof. In certain embodiments, 1, 2, 3, 4, 5, 6, 7, 8, 9 or 10 amino acids of SEQ. ID NO: 15 are replaced by another amino acid.

In further particular embodiments, the VHH antibody is selected from antibody Ma7B01 having a VHH sequence as shown in SEQ. ID NO: 19 or a VHH antibody, which is a variant thereof. In certain embodiments, 1, 2, 3, 4, 5, 6, 7, 8, 9 or 10 amino acids of SEQ. ID NO: 19 are replaced by another amino acid.

In further particular embodiments, the VHH antibody is selected from antibody Ma7C02 having a VHH sequence as shown in SEQ. ID NO: 23 or a VHH antibody, which is a variant thereof. In certain embodiments, 1, 2, 3, 4, 5, 6, 7, 8, 9 or 10 amino acids of SEQ. ID NO: 23 are replaced by another amino acid.

In further particular embodiments, the VHH antibody is selected from antibody Ma7E01 having a VHH sequence as shown in SEQ. ID NO: 27 or a VHH antibody, which is a variant thereof. In certain embodiments, 1, 2, 3, 4, 5, 6, 7, 8, 9 or 10 amino acids of SEQ. ID NO: 27 are replaced by another amino acid.

In further particular embodiments, the VHH antibody is selected from antibody Ma7F02 having a VHH sequence as shown in SEQ. ID NO: 31 or a VHH antibody, which is a variant thereof. In certain embodiments, 1, 2, 3, 4, 5, 6, 7, 8, 9 or 10 amino acids of SEQ. ID NO: 31 are replaced by another amino acid.

In further particular embodiments, the VHH antibody is selected from antibody Ma7D06 having a VHH sequence as shown in SEQ. ID NO: 35 or a VHH antibody, which is a variant thereof. In certain embodiments, 1, 2, 3, 4, 5, 6, 7, 8, 9 or 10 amino acids of SEQ. ID NO: 35 are replaced by another amino acid.

In further particular embodiments, the VHH antibody is selected from antibody Ma7C11 having a VHH sequence as shown in SEQ. ID NO: 39 or a VHH antibody, which is a variant thereof. In certain embodiments, 1, 2, 3, 4, 5, 6, 7, 8, 9 or 10 amino acids of SEQ. ID NO: 39 are replaced by another amino acid.

In further particular embodiments, the VHH antibody is selected from antibody Ma8E03 having a VHH sequence as shown in SEQ. ID NO: 43 or a VHH antibody, which is a variant thereof. In certain embodiments, 1, 2, 3, 4, 5, 6, 7, 8, 9 or 10 amino acids of SEQ. ID NO: 43 are replaced by another amino acid.

In further particular embodiments, the VHH antibody is selected from antibody Ma8G02 having a VHH sequence as shown in SEQ. ID NO: 47 or a VHH antibody, which is a variant thereof. In certain embodiments, 1, 2, 3, 4, 5, 6, 7, 8, 9 or 10 amino acids of SEQ. ID NO: 47 are replaced by another amino acid.

In further particular embodiments, the VHH antibody is selected from antibody Ma8H03 having a VHH sequence as shown in SEQ. ID NO: 51 or a VHH antibody, which is a variant thereof. In certain embodiments, 1, 2, 3, 4, 5, 6, 7, 8, 9 or 10 amino acids of SEQ. ID NO: 51 are replaced by another amino acid.

In further particular embodiments, the VHH antibody is selected from antibody Ma8A05 having a VHH sequence as shown in SEQ. ID NO: 55 or a VHH antibody, which is a variant thereof. In certain embodiments, 1, 2, 3, 4, 5, 6, 7, 8, 9 or 10 amino acids of SEQ. ID NO: 55 are replaced by another amino acid.

In further particular embodiments, the VHH antibody is selected from antibody Ma8D05 having a VHH sequence as shown in SEQ. ID NO: 59 or a VHH antibody, which is a variant thereof. In certain embodiments, 1, 2, 3, 4, 5, 6, 7, 8, 9 or 10 amino acids of SEQ. ID NO: 59 are replaced by another amino acid.

In further particular embodiments, the VHH antibody is selected from antibody Ma8D07 having a VHH sequence as shown in SEQ. ID NO: 63 or a VHH antibody, which is a variant thereof. In certain embodiments, 1, 2, 3, 4, 5, 6, 7, 8, 9 or 10 amino acids of SEQ. ID NO: 63 are replaced by another amino acid.

In further particular embodiments, the VHH antibody is selected from antibody Ma8D08 having a VHH sequence as shown in SEQ. ID NO: 67 or a VHH antibody, which is a variant thereof. In certain embodiments, 1, 2, 3, 4, 5, 6, 7, 8, 9 or 10 amino acids of SEQ. ID NO: 67 are replaced by another amino acid.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well-known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an *E. coli* or a *Bacillus sp.* cell, a yeast cell, particularly a *Pichia* yeast cell, an insect cell, e.g., an Sf-9 cell, or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

### Binding to S. aureus HLA

The VHH antibodies of the present invention bind to an S. *aureus* HLA polypeptide. The inventors have identified VHH antibodies, which bind with high affinity to different epitopes on *S. aureus* HLA, as shown in Table 1, supra.

In the context of the present disclosure the term *"S. aureus* HLA" encompasses *S. aureus* HLA from different strains, e.g., MRSA-resistant strains, such as USA300, USA400 (CC1), USA500 (CC8) or ST80. These and other suitable strains are e.g., described by Diep et al. (2006), Lancet 367, 731-739; Cortes et al. (2017), Mem Inst Oswaldo Cruz, Rio de Janeiro 112, 790-792; Earls et al. (2019), Infection, Genetics and Evolution 69, 117-126; Frisch et al. (2018), mSphere 3, e00571-17; Mairi et al. (2020), Toxins (Basel) 12, 119:

It should be noted, however, that the term *"S. aureus* HLA" also encompasses naturally occurring variants of S. *aureus* HLA polypeptides and genetically modified constructs as described herein.

The inventors have performed their selection and crystallization experiments with HLA shown in SEQ ID NO: 1 and the Octet assays with Cys-HLA following biotinylation at the N-terminal ectopic cysteine with Cys-HLA as shown in SEQ. ID NO: 2.

In certain embodiments, the VHH antibody of the present invention binds to S. *aureus* HLA wherein the binding affinity expressed as dissociation constant K_{D} is about 1 nM or less, of about 0.5 nM or less, or of about 0.1 nM or less. The binding affinity may be determined as described herein in detail in the Examples using the polypeptides of SEQ. ID NO: 1 and 2 as described above.

### HLA neutralization

The VHH antibodies of the present invention are capable of neutralizing *S. aureus* HLA, particularly by inhibiting the hemolysis of blood cells, e.g., erythrocytes, as shown in Table 1, supra.

In certain embodiments, the VHH antibody of the present invention alters the biological properties of HLA, particularly by inhibiting receptor and/or membrane binding of HLA and/or formation of a heptameric pore.

In certain embodiments, the VHH antibody, e.g., Ma8A05 or a competing antibody, binds the hemolysin protomer of HLA and blocks the assembly of the heptameric pore. In further embodiments, the VHH antibody, e.g., Ma7A07 and Ma8D07, or a competing VHH antibody, blocks subunit association. In further embodiments, the VHH antibody, e.g.,Ma7F02 or a competing VHH antibody, inhibits a conformational change that generates the membrane-inserting β-hairpin.

In certain embodiments, the VHH antibody of the present invention neutralizes HLA at a concentration that stoichiometrically corresponds to the concentration of HLA. The neutralization potency may be determined as described herein in detail in the Examples. In Example 3, erythrocyte lysis *in vitro* was observed at an HLA concentration of about 0.5-1 nM. This lysis could be neutralized with an VHH antibody concentration of about 1 nM.

### Stability

For the intended therapeutic application, the anti-HLA antibodies should not only be highly potent in HLA neutralization, but also, they should be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry. The present inventors have identified several thermostable or hyperthermostable VHH antibodies as shown in Table 1, supra.

In a particular embodiment, the invention relates to a VHH antibody, which is stable, particularly thermostable, or hyperthermostable. Preferably, the VHH antibody has a melting point (melting temperature) of at least about 40°C, of at least about 50°C, of at least about 60°C, of at least about 80°C, of at least 90°C or of at least about 95°C, and/or an aggregation temperature of at least about 40°C, of at least about 50°C, of at least about 60°C, of at least 70°C or of at least about 80°C when measured under non-reducing conditions. Melting and aggregation temperatures are determined as described herein.

### Sets of VHH antibodies

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

In particular embodiments, the set of VHH antibodies comprises at least two, e.g., two, three or four complementary VHH antibodies, i.e., VHH antibodies directed against different epitopes of HLA, e.g., a VHH antibody directed against epitope 1, a VHH antibody directed against epitope 2, a VHH antibody directed against epitope 3, and/or a VHH antibody directed against epitope 4 as defined herein.

For example, the set of VHH antibodies comprises at least two, e.g., two, three or four complementary VHH antibodies, selected from at least two, three or four different VHH antibody classes (i), (ii), (iii) and (iv):
(i) a VHH antibody which is selected from VHH antibody Ma7A07 having a VHH sequence as shown in SEQ. ID NO: 3, or a VHH antibody, which competes with the VHH antibody Ma7A07 for the binding to HLA;
(ii) a VHH antibody which is selected from VHH antibody Ma7B01 having a VHH sequence as shown in SEQ. ID NO: 19, or a VHH antibody, which competes with the VHH antibody Ma7B01 for the binding to HLA;
(iii) a VHH antibody which is selected from VHH antibody Ma8A05 having a VHH sequence as shown in SEQ. ID NO: 55, or a VHH antibody which competes with the VHH antibody Ma8A05 for the binding to HLA; and
(iv) a VHH antibody which is selected from VHH antibody Ma8D07 having a VHH sequence as shown in SEQ. ID NO: 63, or a VHH antibody which competes with the VHH antibody Ma8D07 for the binding to HLA.

A particularly preferred set of VHH antibodies includes:
- VHH antibody Ma7A07 having a VHH sequence as shown in SEQ. ID NO: 3,
- VHH antibody Ma7B01 having a VHH sequence as shown in SEQ. ID NO: 19,
- VHH antibody Ma8D07 having a VHH sequence as shown in SEQ. ID NO: 63, and optionally VHH antibody Ma8A05 having a VHH sequence as shown in SEQ. ID NO: 55.

A further particularly preferred set of VHH antibodies includes:
- VHH antibody Ma7A07 having a VHH sequence as shown in SEQ. ID NO: 3,
- VHH antibody Ma7F02 having a VHH sequence as shown in SEQ. ID NO: 31,
- VHH antibody Ma8D07 having a VHH sequence as shown in SEQ. ID NO: 63, and optionally VHH antibody Ma8A05 having a VHH sequence as shown in SEQ. ID NO: 55.

A further particularly preferred set of VHH antibodies includes:
- VHH antibody Ma8E03 having a VHH sequence as shown in SEQ. ID NO: 43,
- VHH antibody Ma7F02 having a VHH sequence as shown in SEQ. ID NO: 31,
- VHH antibody Ma8D07 having a VHH sequence as shown in SEQ. ID NO: 63, and optionally VHH antibody Ma8A05 having a VHH sequence as shown in SEQ. ID NO: 55.

A further particularly preferred set of VHH antibodies includes:
- VHH antibody Ma8E03 having a VHH sequence as shown in SEQ. ID NO: 43,
- VHH antibody Ma7B01 having a VHH sequence as shown in SEQ. ID NO: 19,
- VHH antibody Ma8D07 having a VHH sequence as shown in SEQ. ID NO: 63, and optionally VHH antibody Ma8A05 having a VHH sequence as shown in SEQ. ID NO: 55.

Sets of different VHH antibodies are useful for therapeutic and diagnostic applications as described herein in detail below.

### Monovalent and multivalent VHH antibodies

In certain embodiments, the VHH antibody of the present invention is in a monovalent format, i.e., it has a single binding site for an *S. aureus* polypeptide. In these embodiments, the VHH antibody may be present as such or covalently or non-covalently attached to a heterologous moiety, e.g., a peptidic or non-peptidic moiety.

In further embodiments, the VHH antibody of the present invention is in in a multimeric, e.g., dimeric, or trimeric format. In these embodiments, several VHH antibody units may be covalently or non-covalently attached to each other together via a linker and/or a multimerization, e.g., dimerization or trimerization moiety.

In certain embodiments, the VHH antibody may be covalently or non-covalently conjugated to a heterologous moiety, which is selected from a labeling group, a capture group, or an effector group, and wherein the heterologous moiety is particularly selected from a fluorescence group, biotin, an enzyme such as a peroxidase, phosphatase, or luciferase, a hapten, an affinity tag, or a nucleic acid such as an oligonucleotide. In further embodiments, the heterologous moiety is selected from human serum albumin, an albumin-binding moiety, or an Fc fragment of an immunoglobulin molecule, e.g., IgA, IgD, IgE, IgG, IgM, or a subtype thereof. In still further embodiments, the heterologous moiety is selected from one or several non-peptidic polymer moieties, preferably hydrophilic polymer moieties, such as polyethylene glycol (PEG).

In certain embodiments, the VHH antibody is a homodimeric VHH antibody, wherein a VHH antibody unit is covalently attached to a dimerization moiety, e.g., an immunoglobulin IgG Fc fragment.

In certain embodiments, the VHH antibody is a heterodimeric VHH antibody, particularly a covalently linked VHH heterodimer comprising a first VHH antibody and a second VHH antibody wherein the first VHH antibody and the second VHH antibody bind to different epitopes on HLA.

### Production of VHH antibodies

VHH antibodies including monomeric and multimeric VHH antibodies may be produced as described in WO 2022/023483 and WO 2022/023484, the contents of which are herein incorporated by reference, or by other methods well known in the art.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The nucleic acid molecule may encode a monomeric VHH antibody or a subunit of a multimeric VHH antibody.

In a particular embodiment, the VHH antibody is recombinantly produced in a bacterium, e.g., *E. coli* or *Bacillus.* For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In a further particular embodiment, the VHH antibody is recombinantly produced in a eukaryotic host cell or host organism, preferably in yeast, e.g., in *Pichia pastoris, Saccharomyces cerevisiae, Schizosaccharomyces pombe,* or *Hansenula polymorpha,* or in an animal cell, particularly in an insect cell, e.g., an Sf-9 cell, or a mammalian cell, e.g., human or a hamster cell such as HEK293F, CHO or COS-7. For example, expression in a eukaryotic host cell or host organism, e.g., yeast may involve cytoplasmic and/or periplasmic expression and purification of the VHH antibody from the host cell, or preferably secretion from the host cell and purification of the VHH antibody from the culture medium. In certain embodiments, the nucleic acid sequence encoding the VHH antibody is fused to at least one sequence directing the expression into the culture medium.

### Therapeutic applications

Still a further aspect of the present invention is the use of a VHH antibody as described above in medicine, particularly for therapeutic and/or in vitro or vivo diagnostic use. In certain embodiments, the VHH antibody is used in human medicine.

The VHH antibody of the present invention is useful in the prevention, treatment and/or mitigation of a condition caused by, associated with and/or accompanied by an infection with an HLA-positive *S. aureus.* In certain embodiments, the *S. aureus* is a drug-resistant *S. aureus.*

Exemplary conditions include, but are not limited to a skin infection, pneumonia, bacteremia, sepsis, meningitis, osteomyelitis, and/or endocarditis.

In therapeutic applications, the VHH antibody is administered in an effective amount to a subject in need thereof, particularly to a human subject. The dose will depend on the specific type of agent, e.g., monovalent VHH antibody or multimeric VHH antibody, the type of disease, and the route of administration.

Typically, the VHH antibody is administered as a pharmaceutical composition comprising the active agent and a pharmaceutically acceptable carrier or excipient. Examples of suitable carriers and excipients for formulating antibodies or antibody fragments are well-known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g., by subcutaneous, intramuscular, or intravenous injection or by infusion. In further embodiments, the pharmaceutical composition is administered locally, e.g., topically, orally, nasally, for example by spraying or intrapulmonary, for example by inhalation as an aerosol.

In certain embodiments, treatment of a patient infected by HLA-positive S. *aureus* involves a systemic application of one or several complementary neutralizing anti-HLA VHH antibodies, for example, by route of injection. In the case of pneumonia, inhalation might be a preferred application route. In both cases, the treatment should reduce tissue damage by HLA and the virulence of the pathogen to allow the immune system to clear the infection.

Prophylactic use of anti-HLA VHH antibodies is considered for a subject that is colonized by *S. aureus* or has been exposed or is likely exposed to the pathogen and/or a subject that is immune-compromised, prepared for surgery and/or has suffered injuries that make an infection likely.

Anti-HLA VHH antibodies will be effective also against highly antibiotic-resistant strains, and in an advanced state of infection, e.g., in an advanced state of sepsis, where the toxin's action can no longer be cleared by antibiotic treatment alone.

The VHH antibody may be administered alone as a monotherapy or together, e.g., sequentially, or simultaneously, with a further active agent as a combination therapy, particularly with a further agent that is useful in the prevention, treatment, and/or mitigation of a disorder caused by and/or associated with an infection with *S. aureus* and/or optionally a different bacterium. In certain embodiments, the VHH antibody is administered in combination with an antibiotic against *S. aureus,* e.g., vancomycin, and optionally with a further antibiotic against a different bacterium.

### Diagnostic applications

Diagnostic applications include in vitro methods wherein a VHH antibody or a set of different VHH antibodies is used for detecting HLA in a sample, e.g., in a body fluid such as saliva, sputum, a lung lavage, a swab, blood, serum or plasma, stool samples or in a tissue or biopsy sample.

Diagnostic applications further include in vivo methods wherein a VHH antibody is used for detecting HLA in a subject, particularly in a human patient. For diagnostic applications, the VHH antibody may carry a label for direct detection or used in combination with secondary detection reagents, e.g., biotin/streptavidin, detection antibodies, including conventional antibodies or VHH antibodies, for indirect detection according to established techniques in the art.

The present invention encompasses the use of anti-HLA VHH antibodies for detecting the toxin with high sensitivity. The availability of four complementary epitope classes can be exploited for highly sensitive detection. In certain embodiments, the detection format comprises the binding of at least 2 different VHH antibodies, e.g., 2, 3, or 4 different VHH antibodies to an HLA molecule, particularly the binding of at least 2 different VHH antibodies, e.g., 2, 3, or 4 VHH antibodies each directed against a different epitope. In particular embodiments, the detection comprises the use of a set of 2, 3 or 4 different VHH antibodies as described above.

In particular embodiments, the detection comprises a sandwich assay, e.g., a sandwich ELISA. In such a test format, a first VHH antibody may be immobilized to a solid phase for capturing HLA from the sample, and a second VHH antibody is employed in labelled form for the actual detection. A third and/or fourth VHH antibody can also be used for a prior enrichment step, e.g., by a capture and proteolytic releaseapproach (Frey & Görlich, 2014b; Frey & Görlich, 2014a; Vera Rodriguez et al., 2019). This allows a rapid and sensitive detection of HLA which is highly important in life-threatening conditions.

Further, the present invention is explained in more detail by the following Figures and Examples.

### Figure Legends

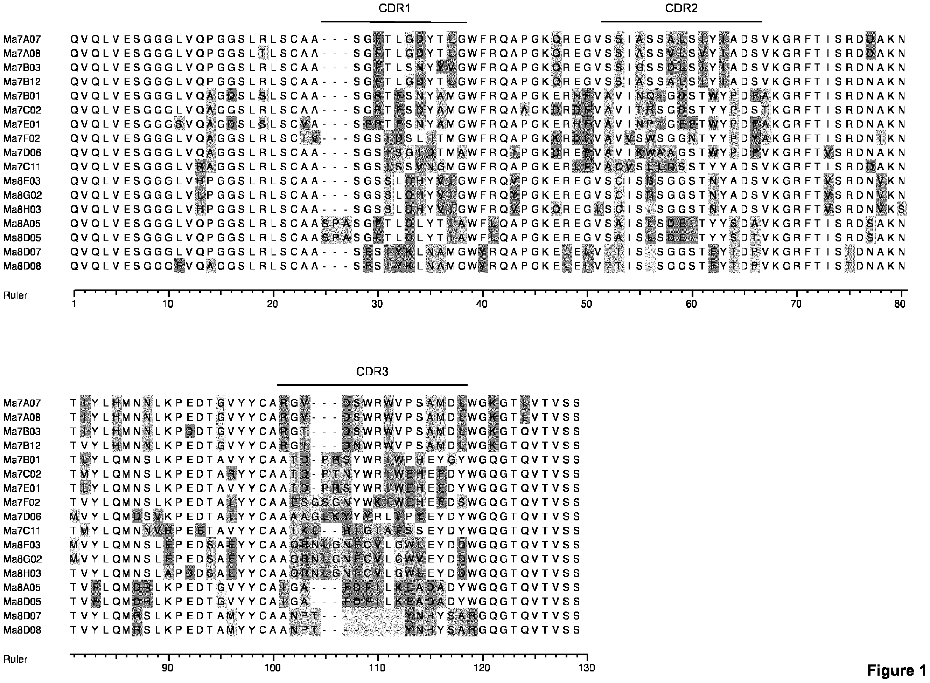

**Figure 1****: Sequence alignment and highlighting of variable regions**
   Figure 1 shows an alignment of VHH sequences from Table 2. Residues that deviate from the consensus are marked by a grey background. The three variable CDR regions are indicated.
**Figure 2****: Affinity measurement of VHH antibodies against HLA**
   Bio-layer interferometry (BLI) experiments were performed for the indicated VHH antibodies on an Octet instrument, with high-precision streptavidin sensors and biotinylated HLA. Black curves represent fits with a mass transport model. The actual datapoints are shown in grey. K_{DS} were derived from a global fit. For details see Example 1.

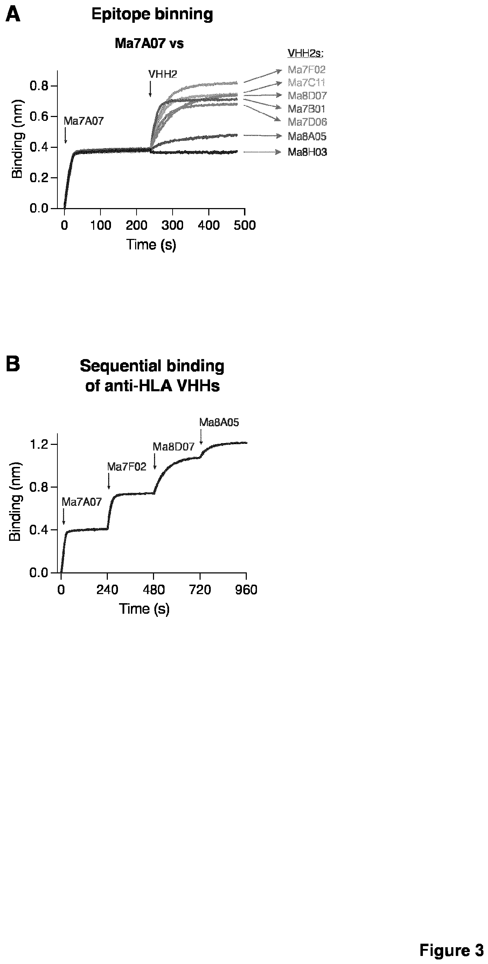

**Figure 3****: Epitope binning of anti-HLA VHH antibodies revealed four distinct HLA-binding sites**
   (A) Epitope-binning experiments were performed pairwise for the indicated VHH antibodies with a biotinylated HLA. When HLA was saturated with Ma7A07, Ma8H03 was not able to bind further, suggesting its binding site was blocked by Ma7A07. Binding of other VHH class antibodies was not affected. Similar pairwise measurements were carried out for all VHH classes.
   (B) Representatives of four distinct epitope VHHs were allowed to bind immobilized HLA sequentially.

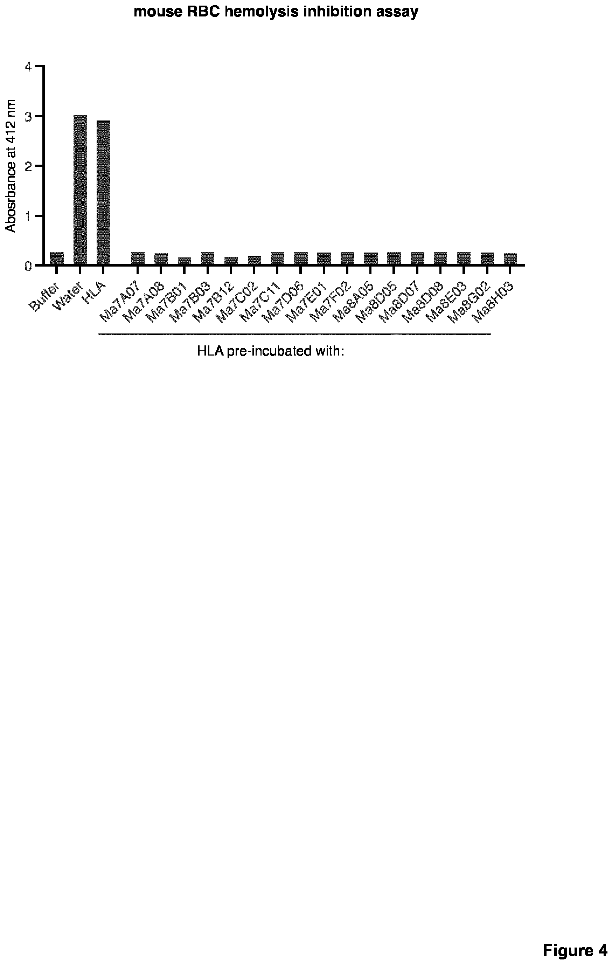

**Figure 4****: Inhibition of HLA-induced hemolysis by VHH antibodies.**
   Mouse erythrocytes were distributed to 96 well plates and incubated with buffer, water or HLA for 3 hours at 37°C followed by 1 hour at 4°C. The plate was centrifuged to pellet the still intact cells, and the supernatants were analyzed on a plate reader for absorption at 412 nm. Lysed cells release their cytoplasmic contents including hemebound hemoglobin, which has a maximum light absorption at 412 nm. Where indicated, HLA was pre-incubated with VHH antibodies.

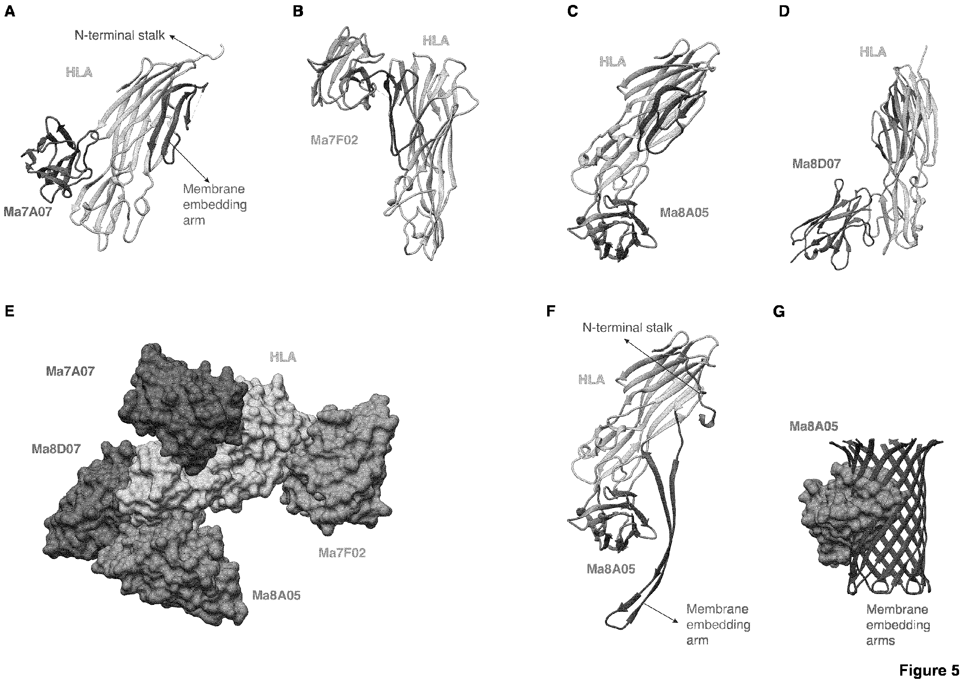

**Figure 5****: High-resolution crystal structures of dimeric VHH•HLA complexes**
   HLA and VHHs were produced in the cytoplasm of *E. coli* NEB Express or NEB Shuffle Express, respectively, and then purified by Ni-chelate chromatography. Dimeric VHH•HLA complexes were formed in solution by mixing HLA and VHH in a 1:1.2 stoichiometry and excess VHHs were removed by gel filtration. The homogenous complexes were crystallized, x-ray diffraction datasets were recorded at the Swiss Light Source synchrotron, and the structures were solved by molecular replacement to a resolution of 2.25 Å and an Rfree of 0.23 for Ma7A07•HLA, 2.20 Å and 0.24 Rfree for Ma7F02•HLA, 2.60 Å and 0.26 Rfree for Ma8A05•HLA, 2.30 Å and 0.23 Rfree for Ma7D07•HLA.
   **A-D)** Crystal structures of the Ma7A07•HLA **(A)**, Ma7F02•HLA **(B)**, Ma8A05•HLA **(C)**, and Ma8D07•HLA **(D)** complexes in ribbon representation. Conformationally important domains of HLA, namely the N-terminal stalk and the membrane-embedding arm, are labelled.
   **E)** Crystal structures shown in **A-D** were superposed by aligning them with respect to HLA, and depicted in surface representation. Note that four VHHs are binding to different portions of the HLA molecule, verifying the epitope-binning experiments.
   **F-G)** In silico-docking of Ma8A05 onto the membrane-bound HLA. Modelling is based on the alignment of HLA in **C** with a single HLA unit in the heptameric HLA structure (PDB ID 3M2L; Banerjee *et al.,* 2010).
   **F)** Ma8A05 bound to single HLA is shown in ribbon representation. The other six HLA protomers are omitted for clarity. Note that Ma8A05 binds to an epitope far from the conformational domains of HLA, and therefore it could recognize HLA in its all forms.
   **G)** Model in **F** is shown with the membrane-embedding domains of all HLA protomers. Major clashes with neighboring HLA protomers indicate that the Ma8A05-bound HLA could not oligomerize into pore-forming heptamer. This explains why this VHH neutralizes toxic HLA effects. The structures of HLA with either Ma7A07, Ma8D07, or Ma7F02 are also all incompatible with the hemolysin forming a multimeric pore.

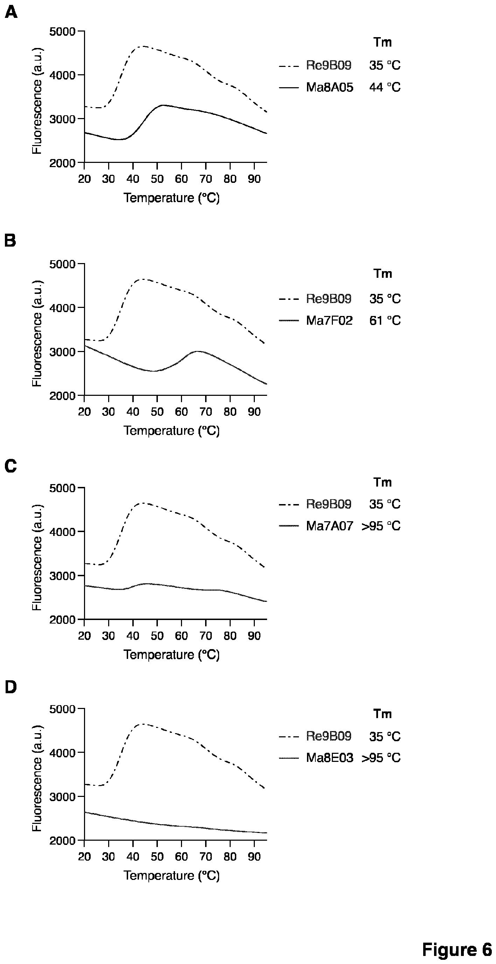

**Figure 6****: Thermostability of VHH antibodies**
   The indicated VHH antibodies were subjected to Differential scanning fluorimetry (DSF), which exploits that thermal unfolding exposes aromatic/hydrophobic residues, which then bind and enhance the fluorescence of the added SYPRO orange dye. Two replicates of each sample were placed onto a Thermal Cycler. The samples were incubated for 5 min at 25°C and then the temperature was increased in 1 °C increments of 45 seconds to 95°C. Fluorescence was measured at the end of each step.

### HLA sequences

>HLA used for hemolysis assays and crystallization (SEQ ID NO: 1) 
ADSDINIKTGTTDIGSNTTVKTGDLVTYDKENGMHKKVFYSFIDDKNHNKKLLVIRTKGTIA
GQYRVYSEEGANKSGLAWPSAFKVQLQLPDNEVAQISDYYPRNSIDTKEYMSTLTYGFNGNV
TGDDTGKIGGLIGANVSIGHTLKYVQPDFKTILESPTDKKVGWKVIFNNMVNQNWGPYDRDS
WNPVYGNQLFMKTRNGSMKAADNFLDPNKASSLLSSGFSPDFATVITMDRKASKQQTNIDVI
YERVRDDYQLHWTSTNWKGTNTKDKWTDRSSERYKIDWEKEEMTN
>Cys-HLA used for Octet assays following biotinylation at the N-terminal ectopic cysteine (SEQ ID NO: 2)

SECGSSGADSDINIKTGTTDIGSNTTVKTGDLVTYDKENGMHKKVFYSFIDDKNHNKKLLVI
RTKGTIAGQYRVYSEEGANKSGLAWPSAFKVQLQLPDNEVAQISDYYPRNSIDTKEYMSTLT
YGFNGNVTGDDTGKIGGLIGANVSIGHTLKYVQPDFKTILESPTDKKVGWKVIFNNMVNQNW
GPYDRDSWNPVYGNQLFMKTRNGSMKAADNFLDPNKASSLLSSGFSPDFATVITMDRKASKQ
QTNIDVIYERVRDDYQLHWTSTNWKGTNTKDKWTDRSSERYKIDWEKEEMTN

### VHH antibody sequences

>Ma7A07 (SEQ ID NO: 3) 
QVQLVESGGGLVQPGGSLRLSCAASGFTLGDYTLGWFRQAPGKQREGVSSIASSALSIYIAD
SVKGRFTISRDDAKNTIYLHMNNLKPEDTGVYYCARGVDSWRWVPSAMDLWGKGTLVTVSS
>Ma7A08 (SEQ ID NO: 7) 
QVQLVESGGGLVQPGGSLTLSCAASGFTLGDYTLGWFRQAPGKQREGVSSIASSVLSVYIAD
SVKGRFTISRDDAKNTIYLHMNNLKPEDTGVYYCARGVDSWRWVPSAMDLWGKGTLVTVSS
>Ma7B03 SEQ ID NO: 11 
QVQLVESGGGLVQPGGSLRLSCAASGFTLSNYYVGWFRQAPGKQREGVSSIGSSDLSIYIAD
SVKGRFTISRDNAKNTIYLHMNNLKPDDTGVYYCARGTDSWRWVPSAMDLWGKGTQVTVSS
>Ma7B12 SEQ ID NO: 15 
QVQLVESGGGLVQPGGSLRLSCAASGFTLGDYTLGWFRQAPGKQREGVSSIASSALSIYIAD
SVKGRFTISRDNAKNTVYLHMNNLKPEDTGVYYCARGIDNWRWVPSAMDLWGKGTQVTVSSG
KTLVTVSS
>Ma7B01 SEQ ID NO: 19 
QVQLVESGGGLVQAGDSLSLSCAASGRTFSNYAMGWFRQAPGKERHFVAVINQIGDSTWYPD
FAKGRFTISRDNAKNTLYLQMNSLKPEDTAVYYCAATDPRSYWRIWPHEYGYWGQGTQVTVS
S
>Ma7C02 (SEQ ID NO: 23) 
QVQLVESGGGLVQAGGSLRLSCAASGRTFSDYAMGWFRQAAGKDRDFVAVITRSGDSTYYPD
STKGRFTISRDNAKNTMYLQMNSLKPEDTARYYCAATDPTNYWRIWEHEFDYWGQGTQVTVS
S
>Ma7E01 SEQ ID NO: 27 
QVQLVESGGGSVQAGDSLSLSCVASERTFSNYAMGWFRQAPGKERHFVAVINPIGEETWYPD
FAKGRFTISRDNAKNTLYLQMNSLKPEDTAVYYCAATDPRSYWRIWEHEFDYWGQGTQVTVS
S
>Ma7F02 (SEQ ID NO: 31) 
QVQLVESGGGLVQAGGSLRLSCTVSGSIDSLHTMGWFRQAPGKKRDFVAVVSWSGGNTYYPD
YAKGRFTISRDNTKNTVYLQMNSLKPEDTAIYYCAAESGSGNYWKIWEHEFDSWGQGTQVTV
SS
>Ma7D06 SEQ ID NO: 35) 
QVQLVESGGGLVQAGGSLRLSCAASGSISGIDTMAWFRQIPGKDREFVAVIKWAAGSTWYPD
FVKGRFTVSRDNAKNMVYLQMDSVKPEDTAIYYCAAAAGEKYYYRLFPYEYDYWGQGTQVTV
SS
>Ma7C11 (SEQ ID NO: 39) 
QVQLVESGGGLVRAGGSLRLSCAASGSISSVNGMGWFRQAPGKERLFVAQVSLLDSSTYYAD
SVKGRFTISRDDAKNTMYLQMNNVRPEETAVYYCAATKLRIGTAFSSEYDYWGQGTQVTVSS
>Ma8E03 (SEQ ID NO: 43) 
QVQLVESGGGLVHPGGSLRLSCAASGSSLDHYVIGWFRQVPGKEREGVSCISRSGGSTNYAD
SVKGRFTVSRDNVKNMVYLQMNSLEPEDSAEYYCAAQRNLGNFCVLGWLEYDDWGQGTQVTV
SS
>Ma8G02 (SEQ ID NO: 47) 
QVQLVESGGGLVLPGGSLRLSCAASGSSLDHYVIGWFRQYPGKEREGVSCISRSGGSTNYAD
SVKGRFTVSRDNVKNMVYLQMNSLEPEDSAEYYCAAQRNLGNFCVLGWVEYDDWGQGTQVTV
SS
>Ma8H03 (SEQ ID NO: 51) 
QVQLVESGGGLVHPGGSLRLSCAASGSSLDHYVIGWFRQVPGKQREGISCISSGGSTNYADS
VKGRFTVSRDNVKSTVYLQMNSLAPDDSAEYYCAAQRNLGNFCVLGWLEYDDWGQGTQVTVS
S
>Ma8A05 SEQ ID NO: 55) 
QVQLVESGGGLVQPGGSLRLSCAASPASGFTLDLYTIAWFLQAPGKEREGVSAISLSDEITY
YSDAVKGRFTISRDSAKNTVFLQMDRLKPEDTGVYYCAIGAFDFILKEADADYWGQGTQVTV
SS
>Ma8D05 SEQ ID NO: 59 
QVQLVESGGGLVQPGGSLRLSCAASPASGFTLDLYTIAWFLQAPGKEREGVSAISLSDEITY
YSDTVKGRFTISRDSAKNTVFLQMDRLKPEDTGVYYCAIGAFDFILKEADADYWGQGTQVTV
SS
>Ma8D07 (SEQ ID NO: 63) 
QVQLVESGGGLVQPGGSLRLSCAASESIYKLNAMGWYRQAPGKELELVTTISSGGSTFYTDP
VKGRFTISTDNAKNTVYLQMRSLKPEDTAMYYCAANPTYNHYSARGQGTQVTVSS
>Ma8D08 SEQ ID NO: 67

QVQLVESGGGFVQAGGSLRLSCAASESIYKLNAMGWYRQAPGKELELVTTISSGGSTFYTDP
VKGRFTISTDNAKNTVYLQMRSLKPEDTAMYYCAANPTYNHYSARGQGTQVTVSS

### Examples

VHH antibodies that inhibit HLA activity, were generated by immunizing two alpacas three times in three weeks intervals with a non-toxic HLA mutant (His61Thr), preparing of immune libraries, and performing phage display using HLA as a bait.

192 selected clones were sequenced and classified by sequence similarity. 17 representatives of all VHH classes were then expressed in *E. coli* and purified (see Figure 1 for specific sequences).

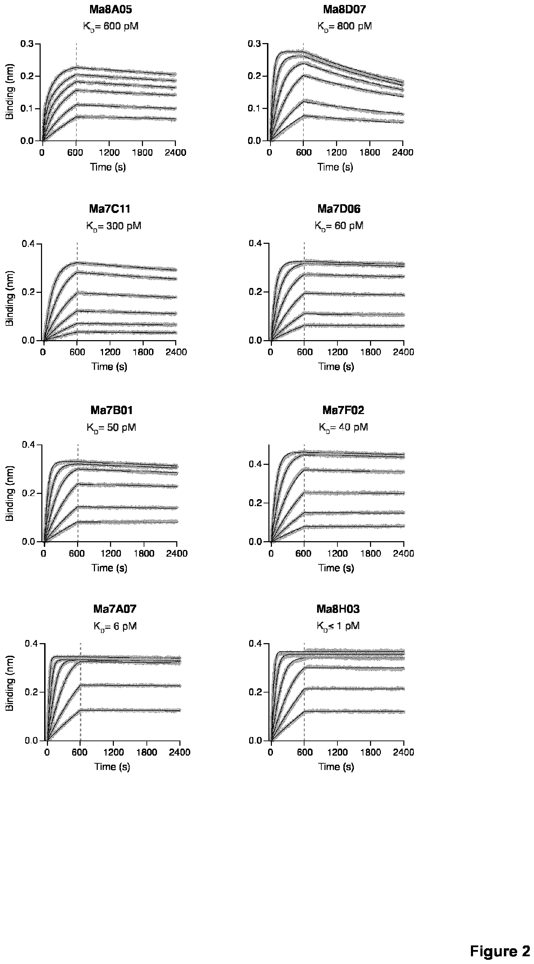

VHH-HLA interactions were analyzed by Bio-Layer Interferometry (BLI; Abdiche et al., 2008). VHH antibodies belonging to Ma8A05 and Ma8D07 classes bound HLA with an intermediate affinity, e.g., with K_{D} values of 600 pM (for Ma8A05) and 800 pM (for Ma8D07; see Figure 2). Other VHH antibodies showed remarkably tight HLA-binding (Table 1 and Figure 2). Ma7B01 and Ma7F02 bound with a K_{D} of ~50 pM. For Ma7A07 and Ma8H03, no dissociation was detected, and K_{D} values of ≤10 pM are estimated.

To assess if and how many complementary binding sites these VHHs have on the HLA molecule, binning experiments were performed using BLI. In these experiments, one VHH antibody was allowed to bind to immobilized HLA, and then the binding of another VHH was monitored. An example is shown in Figure 3. When HLA was completely saturated with Ma7A07, then Ma8H03 (the VHH with the highest affinity for HLA) was not able to bind further, while the binding of other tested VHHs remained unaffected. By repeating this setup with all representative VHH class members, we found that the selected VHH antibodies target four complementary epitopes (Table 1). Figure 3 shows that up to four VHH antibodies can bind HLA simultaneously, provided they are chosen such that they recognize each a different, complementary epitope.

In the following experiments, it was tested whether these VHH antibodies can neutralize HLA. Red blood cells (RBCs) are extremely susceptible to HLA's lytic activity. Moreover, the high concentration of heme (as part of oxygen-carrying hemoglobin) in RBCs makes the detection of cell lysis very straightforward. Therefore, we performed HLA-induced hemolysis experiments using RBCs. We observed that 30 nM HLA lead to complete cell lysis. When tested at a concentration of 100 nM, all anti-HLA VHHs protected cells from hemolysis completely (Figure 4).

To understand the structural basis of the VHH-dependent HLA inhibition, we aimed at crystallizing HLA•VHH complexes. We solved X-ray crystal structures of dimeric HLA complexes of VHH antibodies representing four different epitope binders: Ma7A07, Ma7F02, Ma8A05, and Ma8D07 (Figure 5). The crystallographic analysis verified the fact that these VHHs have four distinct binding sites on HLA. Figure 5 also illustrates as an example the mode of action of Ma8A05: it binds the hemolysin protomer and blocks the assembly of the heptameric pore through clashes with the neighboring subunits. Further analysis revealed that the other VHH antibodies block either subunit association, i.e., Ma7A07 and Ma8D07, or the preceding conformational change that generates the membrane-inserting β-hairpin, i.e., Ma7F02.

### Example 1: Binding of VHH antibodies to HLA

Bio-layer interferometry (BLI) experiments were performed using High Precision Streptavidin biosensors and an Octet RED96e instrument (ForteBio/Sartorius) at 25 °C with Phosphate-Buffered Saline (PBS) pH 7.4, 0.02% (w/v) Tween 20 and 0.1% (w/v) bovine serum albumin (BSA) as assay buffer. For this, HLA had been expressed with an N-terminal ectopic cysteine and biotinylated via attachment of sulfhydrylreactive maleimide-PEG11-biotin (Thermo Scientific, 21911).

Biotinylated HLA was immobilized on biosensors until a wavelength shift/binding signal of 1 nm was reached. The biosensors were dipped into wells containing 40, 20, 10, 5, 2.5 or 1.25 nM VHH for 10 minutes association, and then incubated for 30 minutes with assay buffer for dissociation. Data were reference-subtracted, and curves (gray) were fitted with a mass transport model (Octet Data Analysis HT 12.0 software).

The results are shown in Table 1 and Figure 2.

### Example 2: Identification of complementary HLA epitopes

Epitope binning experiments were performed in a similar set up as described in Example 2. Biotinylated HLA was immobilized on biosensors until a wavelength shift/binding signal of 1 nm was reached. The biosensors were dipped into wells containing 100 nM VHH (VHH1) for 240 seconds, and then incubated for 240 seconds with another VHH (VHH2) at 100 nM concentration. When binding of the VHH2 is blocked in the presence of VHH1, they were considered same epitope binders. By repeating this for all VHH class representatives, we grouped VHH antibodies into four different epitope binders.

The results are shown in Table 1 and Figure 3. Ma7A07, Ma7F02, Ma8D07 and Ma8A05 were able to bind immobilized HLA sequentially.

### Example 3: Erythrocyte hemolysis assays

Erythrocyte stock was freshly prepared from blood sample taken from a CD1 male mouse. 30 nM HLA was diluted in 150 µl of PBS pH 7.4, 0.4% w/v glucose and 25 mM EDTA (assay buffer) in the presence or absence of 100 nM VHH antibodies. After 30 minutes incubation at room temperature, this was added to 50 µL of 4×10⁸ erythrocytes in a well of a standard 96 well plate.

The plate was incubated 3 hours at 37 °C with 180 rpm shaking, followed by 1 hour at 4°C for completion of cell lysis. Afterwards, the plate was centrifuged in a swing out rotor for 10 minutes at 400 xg and 4°C. 150 µL of the supernatant was transferred to another 96 well plate. Analysis was done on a plate reader at 412 nm absorption.

The results are shown in Figure 4. Note that the dilution of erythrocytes in water leads to complete lysis and 30 nM HLA resulted same amount of cell lysis. All VHH antibodies potently inhibited cell lysis and had similar levels as assay buffer.

### Example 4 - Thermostability measurement of VHH antibodies

VHH antibodies were subjected to Differential scanning fluorimetry (DSF), which exploits that thermal unfolding exposes aromatic/hydrophobic residues, which then bind and enhance fluorescence of the added SYPRO orange dye. Assays were performed in a volume of 20 µl, at 1 mg/ml VHH concentration in 50 mM Tris/HCl, 150 mM NaCl (pH 8.0 at 20°C) and 1x dye (diluted from a 5000x stock; Life Technologies). Two replicates of each sample were pipetted in a Hard-Shell^{®} 96-well plate (Bio-Rad). The plate was sealed with transparent MicroSeal^{®} 'B' Seal (Bio-Rad), briefly centrifuged to remove any air bubbles, and placed onto a CFX96 Real-Time System (C1000 Thermal Cycler, BioRad). The samples were incubated for 5 min at 20°C and then the temperature was increased in 1°C increments of 45 seconds to 95°C. Fluorescence was measured at the end of each step with 532 nm excitation and a 555 nm long pass filter. Melting temperatures are defined as the inflection point of the first melting peak.

The results are shown in Table 1 and Figure 6. VHH antibody Re9B09 that was produced in reducing cytosol and hence lacks disulphide bonds melts already at 35°C. Neutralizing VHH antibodies Ma8A05 and Ma7F02 melt similarly at 44°C and 62°C, respectively, while Ma7A07 and Ma8E03 showed only a negligible melting amplitude and thus remained stable throughout 95°C.

### References

Abdiche, Y., Malashock, D., Pinkerton, A., & Pons, J. (2008). Determining kinetics and affinities of protein interactions using a parallel real-time label-free biosensor, the Octet. Anal Biochem, 377(2), 209-217. https://doi.orq/10.1016/j.ab.2008.03.035
Bubeck Wardenburg, J., Patel, R. J., & Schneewind, O. (2007). Surface proteins and exotoxins are required for the pathogenesis of Staphylococcus aureus pneumonia. Infect Immun, 75(2), 1040-1044. https://doi.org/10.1128/|A|.01313-06
Cheung, G. Y. C., Bae, J. S., & Otto, M. (2021). Pathogenicity and virulence of Staphylococcus aureus. Virulence, 12(1), 547-569. https://doi.org/10.1080/21505594.2021.1878688
Frey, S., & Görlich, D. (2014a). Purification of protein complexes of defined subunit stoichiometry using a set of orthogonal, tag-cleaving proteases. J Chromatogr A, 1337, 106-115. https://doi.orq/10.1016/j.chroma.2014.02.030
Frey, S., & Görlich, D. (2014b). A new set of highly efficient, tag-cleaving proteases for purifying recombinant proteins. Journal of Chromatography A, 1337, 95-105. https://doi.org/10.1016/j.chroma.2014.02.029
Ippolito, G., Leone, S., Lauria, F. N., Nicastri, E., & Wenzel, R. P. (2010). Methicillin-resistant Staphylococcus aureus: the superbug. International Journal of Infectious Diseases, 14, S7-S11. https://doi.oro/10.1016/i.ijid.2010.05.003
Kielian, T., Cheung, A., & Hickey, W. F. (2001). Diminished Virulence of an Alpha-Toxin Mutant of <i>Staphylococcus aureus</i> in Experimental Brain Abscesses. Infection and Immunity, 69(11), 6902-6911. https://doi.org/10.1128/iai.69.11.6902-6911.2001
Lodise, T. P., Graves, J., Evans, A., Graffunder, E., Helmecke, M., Lomaestro, B. M., & Stellrecht, K. (2008). Relationship between vancomycin MIC and failure among patients with methicillin-resistant Staphylococcus aureus bacteremia treated with vancomycin. Antimicrob Agents Chemother, 52(9), 3315-3320. https://doi.org/10.1128/AAC.00113-08
Montgomery, C. P., Boyle-Vavra, S., Adem, P. V., Lee, J. C., Husain, A. N., Clasen, J., & Daum, R. S. (2008). Comparison of virulence in community-associated methicillin-resistant Staphylococcus aureus pulsotypes USA300 and USA400 in a rat model of pneumonia. J Infect Dis, 198(4), 561-570. https://doi.org/10.1086/590157
Saleh, M. M., Yousef, N., Shafik, S. M., & Abbas, H. A. (2022). Attenuating the virulence of the resistant superbug Staphylococcus aureus bacteria isolated from neonatal sepsis by ascorbic acid, dexamethasone, and sodium bicarbonate. BMC Microbiology, 22(1). https://doi.org/10.1186/s12866-022-02684-x
Tong, S. Y., Davis, J. S., Eichenberger, E., Holland, T. L., & Fowler, V. G. (2015). Staphylococcus aureus infections: epidemiology, pathophysiology, clinical manifestations, and management. Clin Microbiol Rev, 28(3), 603-661. https://doi.org/10.1128/CMR.00134-14
Turner, N. A., Sharma-Kuinkel, B. K., Maskarinec, S. A., Eichenberger, E. M., Shah, P. P., Carugati, M., Holland, T. L., & Fowler, V. G. (2019). Methicillin-resistant Staphylococcus aureus: an overview of basic and clinical research. Nat Rev Microbiol, 17(4), 203-218. https://doi.org/10.1038/s41579-018-0147-4
Vera Rodriguez, A., Frey, S., & Görlich, D. (2019). Engineered SUMO/protease system identifies Pdr6 as a bidirectional nuclear transport receptor. J Cell Biol, 218(6), 2006-2020. https://doi.orq/10.1083/icb.201812091
Ye, C., Wang, Z., Hu, Y., Deng, C., Liao, L., Sun, L., & Wang, C. (2020). Systematic review and meta-analysis of the efficacy and safety of vancomycin combined with β-lactam antibiotics in the treatment of methicillin-resistant Staphylococcus aureus bloodstream infections. J Glob Antimicrob Resist, 23, 303-310. https://doi.org/10.1016/i.igar.2020.09.024

## Claims

1. A VHH antibody recognizing an *S*. *aureus* hemolysin (HLA),
which competes
(i) with the VHH antibody Ma7A07 having a VHH sequence as shown in SEQ. ID NO: 3 for the binding to HLA; or
(ii) with the VHH antibody Ma7B01 having a VHH sequence as shown in SEQ. ID NO: 19 for the binding to HLA; or
(iii) with the VHH antibody Ma8A05 having a VHH sequence as shown in SEQ. ID NO: 55 for the binding to HLA or
(iv) with the VHH antibody Ma8D07 having a VHH sequence as shown in SEQ. ID NO: 63 for the binding to HLA.

2. A VHH antibody recognizing an *S. aureus* HLA polypeptide, comprising
(a) a CDR3 sequence as shown in SEQ. ID NO: 6, 10, 14, 18, 22, 26, 30, 34, 38, 42, 46, 50, 54, 58, 62, 66 or 70;
(b) a CDR3 sequence, which has an identity of at least 80%, at least 90% or at least 95% to a CDR3 sequence of (a), or
(c) a VHH antibody, which competes with the VHH antibody Ma7A07 having a VHH sequence as shown in SEQ. ID NO: 3 for the binding to HLA, or
which competes with the VHH antibody Ma7B01 having a VHH sequence as shown in SEQ. ID NO: 19 for the binding to HLA, or
which competes with the VHH antibody Ma8A05 having a VHH sequence as shown in SEQ. ID NO: 55 for the binding to HLA, or
which competes with the VHH antibody Ma8D07 having a VHH sequence as shown in SEQ. ID NO: 63 for the binding to HLA.

3. The VHH antibody of claim 1 or 2, comprising
(a) a combination of CDR1, CDR2 and CDR3 sequences as shown in SEQ. ID NO: 4-6, 8-10, 12-14, 16-18, 20-22, 24-26, 28-30, 32-34, 36-38, 40-42, 44-46, 48-50, 52-54, 56-58, 60-62, 64-66, or 68-70,
(b) a combination of CDR1, CDR2 and CDR3 sequences which has an identity of at least 80%, at least 90% or at least 95% to a combination of CDR1, CDR2 and CDR3 sequences of (a), or
(c) a VHH antibody, which competes with the VHH antibody Ma7A07 having a VHH sequence as shown in SEQ. ID NO: 3 for the binding to HLA, or
which competes with the VHH antibody Ma7B01 having a VHH sequence as shown in SEQ. ID NO: 19 for the binding to HLA, or
which competes with the VHH antibody Ma8A05 having a VHH sequence as shown in SEQ. ID NO: 55 for the binding to HLA, or
which competes with the VHH antibody Ma8D07 having a VHH sequence as shown in SEQ. ID NO: 63 for the binding to HLA.

4. The VHH antibody of any one of the preceding claims comprising
(c) a VHH sequence as shown in SEQ. ID NO: 3, 7, 11, 15, 19, 23, 27, 31, 35, 39, 43, 47, 51, 55, 59, 63 or 67;
(d) a sequence, which has an identity of at least 80%, at least 90%, at least 95% or at least 99% to a VHH sequence of (a), or
(c) a VHH antibody, which competes with the VHH antibody Ma7A07 having a VHH sequence as shown in SEQ. ID NO: 3 for the binding to HLA, or
which competes with the VHH antibody Ma7B01 having a VHH sequence as shown in SEQ. ID NO: 19 for the binding to HLA, or
which competes with the VHH antibody Ma8A05 having a VHH sequence as shown in SEQ. ID NO: 55 for the binding to HLA, or
which competes with the VHH antibody Ma8D07 having a VHH sequence as shown in SEQ. ID NO: 63 for the binding to HLA.

5. The VHH antibody of any one of the preceding claims, wherein the binding affinity expressed as dissociation constant K_{D} to HLA is about 1 nM or less, about 100 pM or less, about 50 pM or less, about 20 pM or less or about 10 pM or less.

6. The VHH antibody of any one of the preceding claims which neutralizes HLA.

7. The VHH antibody of any one of the preceding claims, which is stable, particularly thermostable, or hyperthermostable and which has a melting temperature of at least about 50°C, of at least about 60°C, of at least 80°C or of at least about 95°C when measured under non-reducing conditions.

8. The VHH antibody of any one of the preceding claims, which is selected from:
(i) a VHH antibody Ma7A07 having a VHH sequence as shown in SEQ. ID NO: 3 or a VHH antibody, which is a variant thereof;
(ii) a VHH antibody, which is selected from VHH antibody Ma7A08 having a VHH sequence as shown in SEQ. ID NO: 7 or a VHH antibody, which is a variant thereof;
(iii) The VHH antibody, which is selected from VHH antibody MaB03 having a VHH sequence as shown in SEQ. ID NO: 11 or a VHH antibody, which is a variant thereof;
(iv) a VHH antibody, which is selected from VHH antibody Ma7B12 having a VHH sequence as shown in SEQ. ID NO: 15 or a VHH antibody, which is a variant thereof;
(v) a VHH antibody, which is selected from VHH antibody Ma7B01 having a VHH sequence as shown in SEQ. ID NO: 19 or a VHH antibody, which is a variant thereof;
(vi) a VHH antibody, which is selected from VHH antibody Ma7C02 having a VHH sequence as shown in SEQ. ID NO: 23 or a VHH antibody, which is a variant thereof;
(vii) a VHH antibody, which is selected from VHH antibody Ma7E01 having a VHH sequence as shown in SEQ. ID NO: 27 or a VHH antibody, which is a variant thereof;
(viii) a VHH antibody, which is selected from VHH antibody Ma7F02 having a VHH sequence as shown in SEQ. ID NO: 31 or a VHH antibody, which is a variant thereof;
(ix) a VHH antibody, which is selected from VHH antibody Ma7D06 having a VHH sequence as shown in SEQ. ID NO: 35 or a VHH antibody, which is a variant thereof;
(x) a VHH antibody, which is selected from VHH antibody Ma7C11 having a VHH sequence as shown in SEQ. ID NO: 39 or a VHH antibody, which is a variant thereof;
(xi) a VHH antibody, which is selected from VHH antibody Ma8E03 having a VHH sequence as shown in SEQ. ID NO: 43 or a VHH antibody, which is a variant thereof;
(xii) a VHH antibody, which is selected from VHH antibody Ma8G02 having a VHH sequence as shown in SEQ. ID NO: 47 or a VHH antibody, which is a variant thereof;
(xiii) a VHH antibody, which is selected from VHH antibody Ma8H03 having a VHH sequence as shown in SEQ. ID NO: 51 or a VHH antibody, which is a variant thereof;
(xiv) a VHH antibody, which is selected from VHH antibody Ma8A05 having a VHH sequence as shown in SEQ. ID NO: 55 or a VHH antibody, which is a variant thereof;
(xv) a VHH antibody, which is selected from VHH antibody Ma8D05 having a VHH sequence as shown in SEQ. ID NO: 59 or a VHH antibody, which is a variant thereof;
(xvi) a VHH antibody, which is selected from VHH antibody Ma8D07 having a VHH sequence as shown in SEQ. ID NO: 63 or a VHH antibody, which is a variant thereof, or
(xvii) a VHH antibody, which is selected from VHH antibody Ma8D08 having a VHH sequence as shown in SEQ. ID NO: 67 or a VHH antibody, which is a variant thereof.

9. A set of two or more different VHH antibodies recognizing HLA, comprising at least one VHH antibody of any of claims 1-8, particularly comprising at least two VHH antibodies selected from at least two different VHH antibody classes (i), (ii), (iii) and (iv) as follows:
(i) a VHH antibody which is selected from VHH antibody Ma7A07 having a VHH sequence as shown in SEQ. ID NO: 3, or a VHH antibody, which competes with the VHH antibody Ma7A07 for the binding to HLA;
(ii) a VHH antibody which is selected from VHH antibody Ma7B01 having a VHH sequence as shown in SEQ. ID NO: 19, or a VHH antibody, which competes with the VHH antibody Ma7B01 for the binding to HLA;
(iii) a VHH antibody which is selected from VHH antibody Ma8A05 having a VHH sequence as shown in SEQ. ID NO: 55, or a VHH antibody which competes with the VHH antibody Ma8A05 for the binding to HLA; and
(iv) a VHH antibody which is selected from VHH antibody Ma8D07 having a VHH sequence as shown in SEQ. ID NO: 63, or a VHH antibody which competes with the VHH antibody Ma8D07 for the binding to HLA.

10. The VHH antibody of any one of claims 1-8 or the set of claim 9 for use in medicine, particularly for use in the prevention, treatment and/or mitigation of a condition caused by, associated with and/or accompanied by an infection with an HLA-positive *S. aureus,* wherein the condition is particularly selected from a skin infection, pneumonia, bacteremia, sepsis, meningitis, osteomyelitis, and/or endocarditis.

11. The VHH antibody of any one of claims 1-8 or the set of claim 9 for the use of claim 10 in a human subject.

12. The VHH antibody of any one of claims 1-8 or the set of claim 9 for the use of any one of claims 10-11, which is administered as a monotherapy or which is administered sequentially and/or simultaneously as a combination therapy with at least one further active agent, e.g., in combination with an antibiotic against *S. aureus,* e.g., vancomycin, and optionally with a further antibiotic against a different bacterium.

13. A pharmaceutical combination comprising as an active agent a VHH antibody of any one of claims 1-8 or the set of claim 9, and a pharmaceutically acceptable carrier.

14. Use of the VHH antibody of any one of claims 1-8 or the set of claim 9 for detecting HLA in a sample, particularly in a biological sample.

15. The use of claim 14, wherein the detection comprises the binding of at least 2 different VHH antibodies to an HLA molecule, particularly .the binding of at least 2 different VHH antibodies, e.g., 2, 3, or 4 VHH antibodies each directed against a different epitope, to an HLA molecule.
